# EUROPEAN PATENT APPLICATION

(11) **EP 4 420 581 A1**
(43) Date of publication of application: **28.08.2024**
(21) Application number: 22884104.5
(22) Date of filing: 21.10.2022
(51) Int. Cl.: A47L 7/00, A47L 9/28, A01M 1/22, A01M 1/20, A01N 25/02, A01N 25/34, A01N 53/00, A01N 31/02

(54) **CLEANER STATION**

(30) Priority: 21.10.2021 KR 20210140688; 21.10.2021 KR 20220061995; 21.10.2021 KR 20220061996
(71) Applicant: LG Electronics Inc., Yeongdeungpo-gu Seoul 07336 (KR)
(72) Inventor: YANG, Ingyu, Seoul 08592 (KR); RYU, Jungwan, Seoul 08592 (KR); SHIN, Jinhyouk, Seoul 08592 (KR); JEONG, Yeonghan, Seoul 08592 (KR); KIM, Misol, Seoul 08592 (KR)
(74) Representative: Ter Meer Steinmeister & Partner
(86) International application number: PCT/KR2022/016189
(87) International publication number: WO 2023/068882

(57) **Abstract**

The present disclosure relates to a cleaner station including a suction flow path having an inlet end configured to communicate with a dust bin of a cleaner, a dust collecting part connected to an outlet end of the suction flow path and configured to capture dust in the dust bin of the cleaner, and a spray module disposed at one side of the dust collecting part and configured to spray at least any one of the sterilization gas and particulates into the dust collecting part to kill insects existing in the dust collecting part, in which the spray module sprays the sterilization gas, which contains ozone or ion particles, into the dust collecting part, to kill insects existing in the dust collecting part.

## Description

### [Technical Field]

The present disclosure relates to a cleaner station, and more particularly, to a cleaner station configured to capture dust in a cleaner.

### [Background Art]

In general, a cleaner refers to an electrical appliance that draws in small garbage or dust by sucking air using electricity and fills a dust bin provided in a product with the garbage or dust. Such a cleaner is generally called a vacuum cleaner.

The cleaners may be classified into a manual cleaner which is moved directly by a user to perform a cleaning operation, and an automatic cleaner which performs a cleaning operation while autonomously traveling. Depending on the shape of the cleaner, the manual cleaners may be classified into a canister cleaner, an upright cleaner, a handy cleaner, a stick cleaner, a robot cleaner, and the like.

The canister cleaners were widely used in the past as household cleaners. However, recently, there is an increasing tendency to use the handy cleaner and the stick cleaner in which a dust bin and a cleaner main body are integrally provided to improve convenience of use.

In the case of the canister cleaner, a main body and a suction port are connected by a rubber hose or pipe, and in some instances, the canister cleaner may be used in a state in which a brush is fitted into the suction port.

The handy cleaner (hand vacuum cleaner) has maximized portability and is light in weight. However, because the handy cleaner has a short length, there may be a limitation to a cleaning region. Therefore, the handy cleaner is used to clean a local place such as a desk, a sofa, or an interior of a vehicle.

A user may use the stick cleaner while standing and thus may perform a cleaning operation without bending his/her waist. Therefore, the stick cleaner is advantageous for the user to clean a wide region while moving in the region. The handy cleaner may be used to clean a narrow space, whereas the stick cleaner may be used to clean a wide space and also used to a high place that the user's hand cannot reach. Recently, modularized stick cleaners are provided, such that types of cleaners are actively changed and used to clean various places.

Recent cleaners have been miniaturized to maximize portability. For this reason, recently produced cleaners have dust bins with small capacities for storing collected dust, which inconveniences the user because the user needs to empty the dust bin frequently.

A cleaner station has been developed to cope with the inconvenience. The cleaner station refers to a device configured to accommodate the cleaner, which has completed the cleaning operation, and to suck the dust collected by the cleaner. In general, the cleaner station is equipped with a dust collecting part much larger in size than the dust bin of the cleaner. That is, the dust collected by the cleaner is continuously captured in the dust collecting part of the cleaner station, and the user empties the dust collecting part of the cleaner station. The configuration using the cleaning station has an advantage in that the number of times the user empties the dust bin significantly decreases in comparison with a case in which the user frequently empties the dust bin of the cleaner.

U.S. Patent No. 9,924,846 will be described as a preceding document. The preceding document related to a discharge station.

The preceding document discloses that when a robot cleaner is coupled to the discharge station, the discharge station sucks dust from a dust bin of the robot cleaner and collects the dust in a bag. A latex membrane is disposed in a connection portion between an inlet of the bag and a suction tube. When an upper cover is closed after the bag is installed in the discharge station, the suction tube is inserted into the bag, the suction tube penetrates the latex membrane, and the latex membrane is deformed. Therefore, sealability is maintained between the suction tube and the bag.

According to the preceding document, because the latex membrane is always installed in the inlet of the bag and the suction tube is inserted while penetrating the latex membrane, it can be seen that the latex membrane has a hollow portion penetrated by the suction tube. For this reason, there may occur a problem in that fine dust scatters from the hollow portion of the latex membrane, flying insects escape, or offensive odor is diffused when the bag is separated from the discharge station.

In particular, as the frequency of emptying the dust collecting part of the cleaner station significantly increases, the collected dust is stored in the dust collecting part over a long period of time. In this case, because an internal space of the dust collecting part is maintained with a moderate temperature and appropriate humidity, bacteria or insects may easily grow. In fact, there are many complaints related to the explosive breeding of aphids such as spider mites in the cleaner station, which makes it unpleasant for the user of the cleaner.

### [Disclosure]

### [Technical Problem]

An object to be achieved by the present disclosure is to provide a cleaner station capable of killing insects that may exist in a dust collecting part in the cleaner station configured to capture dust collected in a dust bin of a cleaner.

Another object to be achieved by the present disclosure is to provide a cleaner station capable of preventing a sterilization gas sprayed into a dust collecting part from leaking to the outside of a cleaner station and harming a user.

Still another object to be achieved by the present disclosure is to provide a cleaner station capable of preventing water from penetrating into a spray module, which is an electronic device, during a process of cleanly cleaning a dust collecting body by using water or the like.

Yet another object to be achieved by the present disclosure is to provide a cleaner station capable of preventing sprayed ions or ozone from leaking to the outside.

Technical problems of the present disclosure are not limited to the aforementioned technical problems, and other technical problems, which are not mentioned above, may be clearly understood by those skilled in the art from the following descriptions.

### [Technical Solution]

In order to achieve the above-mentioned objects, a cleaner station according to an embodiment of the present disclosure includes: a suction flow path having an inlet end configured to communicate with a dust bin of a cleaner; a dust collecting part connected to an outlet end of the suction flow path and configured to capture dust in the dust bin of the cleaner; and a spray module disposed at one side of the dust collecting part and configured to spray at least any one of the sterilization gas and particulates into the dust collecting part to kill insects existing in the dust collecting part.

The dust collecting part may include: a dust collecting part casing configured to define a space therein; and a dust collecting body separably installed in the dust collecting part casing. In this case, the spray module may be installed at one side of the dust collecting part casing and spray at least any one of the sterilization gas and particulates into the dust collecting body.

When the dust collecting body is installed in the dust collecting part casing, at least a part of the spray module may be inserted into the dust collecting body.

The spray module may include a guide sealer disposed at the outlet end and configured to fill a gap between the outlet end and the dust collecting part. In this case, the dust collecting part may include a spray module insertion hole penetrated by the spray module, and a length from one end to the other end of the guide sealer may be longer than a length from one end to the other end of the spray module insertion hole. Alternatively, the guide sealer may include: a non-deformable portion extending outward from the spray guide and disposed adjacent to the dust collecting part; and a deformable portion further extending outward from an end of the non-deformable portion and configured to be at least partially deformed by being tightly attached to the dust collecting part.

The spray module may include: a sterilization gas generator configured to produce the sterilization gas; and a spray guide disposed at an outlet end of the sterilization gas generator and configured to guide the sterilization gas into the dust collecting part.

The dust collecting part may include: a dust collecting part casing configured to define a space therein; and a dust collecting body separably installed in the dust collecting part casing, the sterilization gas generator may be disposed in the dust collecting part casing, the spray guide may extend toward the dust collecting body, and an outlet end of the spray guide may be inserted into the dust collecting body.

A direction in which the sterilization gas is sprayed by the spray module may be parallel to a direction in which air is introduced into the outlet end of the suction flow path.

In order to achieve the above-mentioned objects, a cleaner station according to an embodiment of the present disclosure includes: a suction flow path having an inlet end configured to communicate with a dust bin of a cleaner; a dust collecting part connected to an outlet end of the suction flow path and configured to capture dust in the dust bin of the cleaner; and a spray module disposed at one side of the dust collecting part and configured to spray at least any one of the sterilization gas and particulates into the dust collecting part to kill insects existing in the dust collecting part. An operation of the spray module may be stopped for a preset reference pause time or longer when the spray module operates for a preset reference operation time.

The cleaner station may further include a dust collecting motor configured to generate an airflow in the suction flow path. In this case, when there is a dust suction instruction for capturing dust in the dust bin of the cleaner, the dust collecting motor may operate after the reference pause time elapses.

The cleaner station may further include: a dust collecting motor configured to generate an airflow in the suction flow path, in which the spray module alternately operates and stops operating while the dust collecting motor does not operate.

Other detailed matters of the exemplary embodiment are included in the detailed description and the drawings.

### [Advantageous Effects]

The cleaner station of the present disclosure has one or more of the following effects.

First, the spray module is disposed at one side of the dust collecting part and sprays the sterilization gas, which contains ozone or ion particles, into the dust collecting part, which kills insects existing in the dust collecting part.

Second, the spray module includes a guide sealer disposed at the outlet end and configured to fill the gap between the outlet end and the dust collecting part, which prevents the sterilization gas from leaking to the outside of the housing.

Third, when the dust collecting body is installed in the dust collecting part casing, at least a part of the spray module is inserted into the dust collecting body, which makes it easy to detach the dust collecting body, separate the dust collecting body from the spray module, and clean the dust collecting body.

Fourth, in case that there is the dust suction instruction, the reference pause time needs to elapse before the dust collecting motor operates. Therefore, the ion or ozone is sufficiently decomposed for the reference pause time, which prevents the ion or ozone from leaking to the outside in accordance with the operation of the dust collecting motor.

Fifth, the spray module alternately operates for the reference operation time and stops operating for the reference pause time while the dust collecting motor does not operate, which prevents breeding of insects in the dust collecting part while the dust collecting motor does not operate.

The effects of the present disclosure are not limited to the aforementioned effects, and other effects, which are not mentioned above, will be clearly understood by those skilled in the art from the claims.

### [Description of Drawings]

FIG. 1 is a perspective view of a cleaner station according to the present disclosure.
FIG. 2 is a view illustrating a suction flow path and a dust collecting part disposed in FIG. 1.
FIG. 3 is an exploded view of the dust collecting part according to the present disclosure.
FIG. 4 is an exploded view of a spray module according to the present disclosure.
FIG. 5 is a right-side cross-sectional side view illustrating the dust collecting part according to the present disclosure and illustrating a state in which a sterilization gas is sprayed.
FIG. 6 is a view illustrating a state made before a dust collecting body is fixed to a dust collecting part casing.
FIG. 7 is a view illustrating a state made after the dust collecting body is fixed to the dust collecting part casing.
FIG. 8 is a view illustrating an operating process of the spray module in the case of the presence of a dust suction instruction.
FIG. 9 is a view illustrating an operating process of the spray module in the case of the absence of a dust suction instruction.

### [Mode for Invention]

Advantages and features of the present disclosure and methods of achieving the advantages and features will be clear with reference to embodiments described in detail below together with the accompanying drawings. However, the present disclosure is not limited to the embodiments disclosed herein but will be implemented in various forms. The embodiments of the present disclosure are provided so that the present disclosure is completely disclosed, and a person with ordinary skill in the art can fully understand the scope of the present disclosure. The present disclosure will be defined only by the scope of the appended claims. Throughout the specification, the same reference numerals denote the same constituent elements.

Hereinafter, the present disclosure will be described with reference to the drawings for explaining a cleaner station 100 according to embodiment of the present disclosure.

A cleaner system is divided into a cleaner 200 and the cleaner station 100. The cleaner is a movable component, and the cleaner sucks dust present in a cleaning region while moving and collects the dust in a dust bin provided in the cleaner. The cleaner 200 is a fixed, non-movable component, and the cleaner station 100 sucks dust from the cleaner 200, which has completed the cleaning operation, and collects the dust in dust collecting parts 140 and 240 provided in the cleaner station 100.

The configuration in which the cleaner 200 is coupled to the cleaner station 100 may include a configuration in which the cleaner is fixed and coupled mechanically to the cleaner station 100, a configuration in which a terminal of the cleaner and a terminal of the cleaner station 100 come into contact with each other and are electrically coupled, and a configuration in which a dust bin of the cleaner and the dust collecting parts 140 and 240 of the cleaner station 100 communicate with each other so that air flows therebetween.

The cleaner 200 may mean a cleaner configured to be manually operated by the user. For example, the cleaner 200 may mean a handy cleaner or a stick cleaner.

The cleaner 200 may include a main body 210. The main body 210 may include a main body housing 211, a suction part 212, a dust separating part 213, a suction motor 214, an air discharge cover 215, a handle 216, an extension part 217, and an operating part 218.

The main body housing 211 may define an external appearance of the cleaner 200. The main body housing 211 may provide a space that may accommodate the suction motor 214 and a filter (not illustrated) therein. The main body housing 211 may be formed in a shape similar to a cylindrical shape.

The suction part 212 may protrude outward from the main body housing 211. For example, the suction part 212 may be formed in a cylindrical shape. The suction part 212 may communicate with an extension tube 250. The suction part 212 may provide a flow path in which air containing dust may flow.

The dust separating part 213 may communicate with the suction part 212. The dust separating part 213 may separate dust sucked into the dust separating part 213 through the suction part 212. The dust separating part 213 may communicate with a dust bin 220.

The dust separating part 213 may be a cyclone part capable of separating dust using a cyclone flow.

The suction motor 214 may generate a suction force for sucking air. The suction motor 214 may be accommodated in the main body housing 211. The suction motor 214 may generate the suction force while rotating. For example, the suction motor 214 may be formed in a shape similar to a cylindrical shape.

The air discharge cover 215 may be disposed in the main body housing 211. The air discharge cover 215 may accommodate the filter for filtering air. For example, an HEPA filter may be accommodated in the air discharge cover 215.

The handle 216 may be grasped by a user. The handle 216 may be disposed rearward of the suction motor 214. For example, the handle 216 may be formed in a shape similar to a cylindrical shape. Alternatively, the handle 216 may be formed in a curved cylindrical shape. The handle 216 may be disposed at a predetermined angle with respect to the main body housing 211, the suction motor 214, or the dust separating part 213.

The extension part 217 may extend from the handle 216 toward the main body housing 211. At least a part of the extension part 217 may extend in a horizontal direction.

The operating part 218 may be disposed on the handle 216. The operating part 218 may be disposed on an inclined surface formed in an upper region of the handle 216. The user may input an instruction to operate or stop the first cleaner 200 through the operating part 218.

The cleaner 200 may include the dust bin 220. The dust bin 220 may communicate with the dust separating part 213. The dust bin 220 may store the dust separated by the dust separating part 213.

The cleaner station 100 is a component coupled to the cleaner 200 and configured to charge the cleaner, suck the dust collected in the dust bin of the cleaner, and safely store the cleaner that has completed the cleaning operation.

The cleaner station 100 includes a housing 110. The housing 110 defines an external shape of the cleaner station 100 and provides a space in which other components are accommodated. The housing 110 may be provided in the form of a column including one or more outer wall surfaces. For example, the housing 110 may be formed in a shape similar to a quadrangular column.

A coupling part 120 is disposed in the housing 110. The coupling part 120 is a component configured to support the cleaner when the cleaner is coupled to the cleaner station 100.

An inlet end of a suction flow path 130 communicates with the dust bin (not illustrated) of the cleaner, and an outlet end of the suction flow path 130 communicates with an inlet pipe 144 of the dust collecting part 140. The suction flow path 130 is a pipe or tube having a hollow portion so that air containing dust flows in the suction flow path 130.

A dust collecting motor 150 is disposed in the housing 110. The dust collecting motor 150 generates negative pressure to generate an airflow in the suction flow path 130 or a discharge flow path (not illustrated). The dust collecting motor 150 communicates with the suction flow path 130 and the discharge flow path (not illustrated).

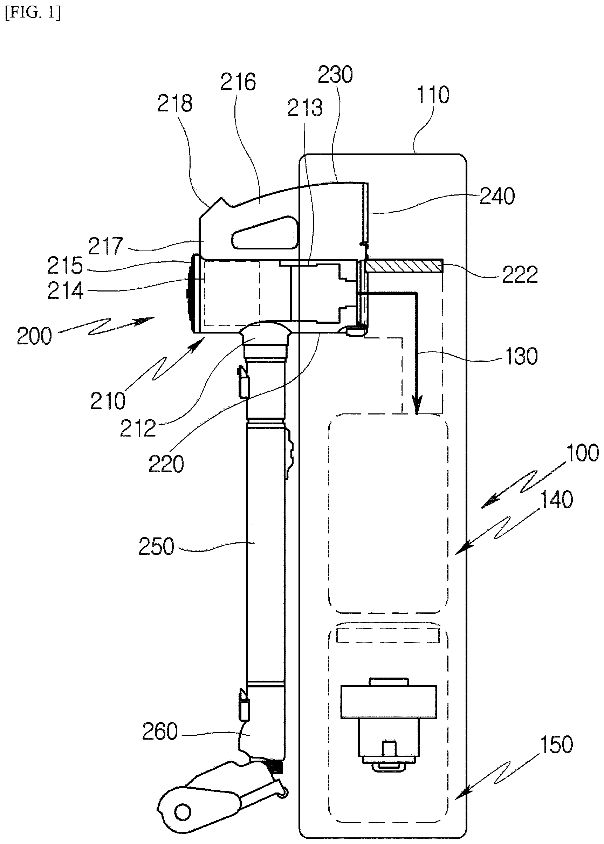

With reference to FIG. 1, the dust collecting motor 150 may be disposed at a downstream side of the dust collecting part 140. Alternatively, the dust collecting motor 150 may be disposed below the dust collecting part 140.

A filter (not illustrated) is disposed in the housing 110. For example, the filter may be disposed between the dust collecting part 140 and the dust collecting motor 150.

The dust collecting part 140 is a component configured to capture dust in the dust bin of the cleaner. The dust collecting part 140 may be accommodated in the housing 110 of the cleaner station 100.

The types of dust collecting parts 140 are broadly classified into a bag type dust collecting part and a bagless type dust collecting part. The bag type dust collecting part 140 is equipped with a separate bag, the bag captures dust, and a user separates only the bag from the cleaner station 100 and discards the bag. In contrast, the bagless type dust collecting part 140 is not equipped with a separate bag, the dust collecting part 140 captures dust, and a user separates the dust collecting part 140 from the cleaner station and eliminates the dust.

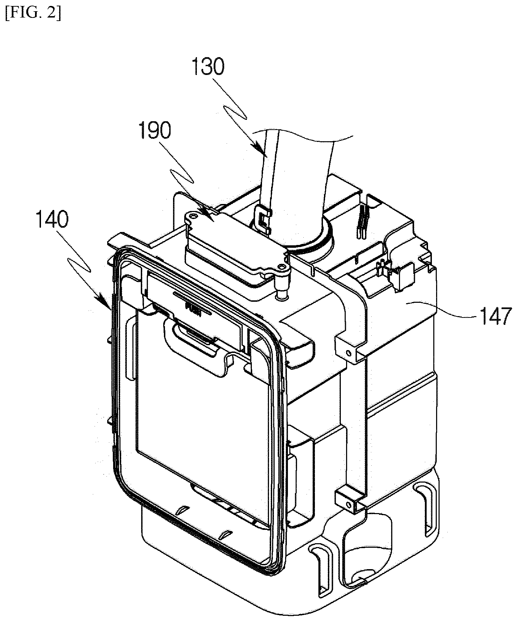

FIGS. 1 to 7 illustrate the bagless type dust collecting part 140. However, the present disclosure is not limited thereto. The dust collecting part 140 may be a bag type dust collecting part or a bagless type dust collecting part.

The dust collecting part 140 according to the present disclosure is disposed separably from the cleaner station 100. The dust collecting part 140 may separate from the cleaner station 100, the collected dust may be removed, and the interior of the dust collecting part 140 may be cleaned. When the dust collecting part 140 is separated from the cleaner station 100, the suction flow path 130 and dust inlet ports 1412, 1422, and 1432 are disconnected. Therefore, there may occur a problem in that insects may escape to the outside, dust may scatter, and offensive odor may be diffused through the dust inlet ports 1412, 1422, and 1432. Therefore, in order to prevent the above-mentioned problem, the dust collecting part 140 according to the present disclosure is equipped with an inlet pipe cover 145 to prevent the problem in that insects escape to the outside, dust scatters, or offensive odor is diffused through the dust inlet ports 1412, 1422, and 1432.

The dust collecting part 140 will be described with reference to FIGS. 1 to 7. The dust collecting part 140 includes a dust collecting body 141, an outer upper plate 142, an inner upper plate 143, and the inlet pipe 144. The dust collecting body 141, the outer upper plate 142, and the inner upper plate 143 are disposed in a dust collecting part casing 147.

The dust collecting part casing 147 defines an external shape of the dust collecting part 140 and defines a space in which the components of the dust collecting part 140 are accommodated.

A front side of the dust collecting part casing 147 is opened, and the components of the dust collecting part 140 may be inserted through the front side of the dust collecting part casing 147.

The dust collecting part casing includes a spray module installation part 1471. The spray module installation part 1471 defines a space in which the spray module is installed. The spray module installation part 1471 may be provided on an upper wall of the dust collecting part casing 147.

The dust collecting part casing 147 includes a dust inlet port 1472. The dust inlet port 1472 is a hole formed through one side wall of the dust collecting part casing 147 in an upward/downward direction. The dust inlet port 1472 of the dust collecting part casing 147 communicates with the dust inlet port 1421 of the outer upper plate 142, the dust inlet port 1432 of the inner upper plate 143, and the dust inlet port 1412 of the dust collecting body 141. The suction flow path 130 is inserted into the dust inlet port 1472 of the dust collecting part casing 147.

The dust collecting body 141 defines an external shape of the dust collecting part 140 and has an internal space in which dust may be collected.

The dust collecting body 141 is separably coupled in the housing 110 of the cleaner station 100. Specifically, the dust collecting body 141 is inserted through a front side of the housing 110 of the cleaner station 100 and coupled to or separated from the housing 110 or the dust collecting part casing 147.

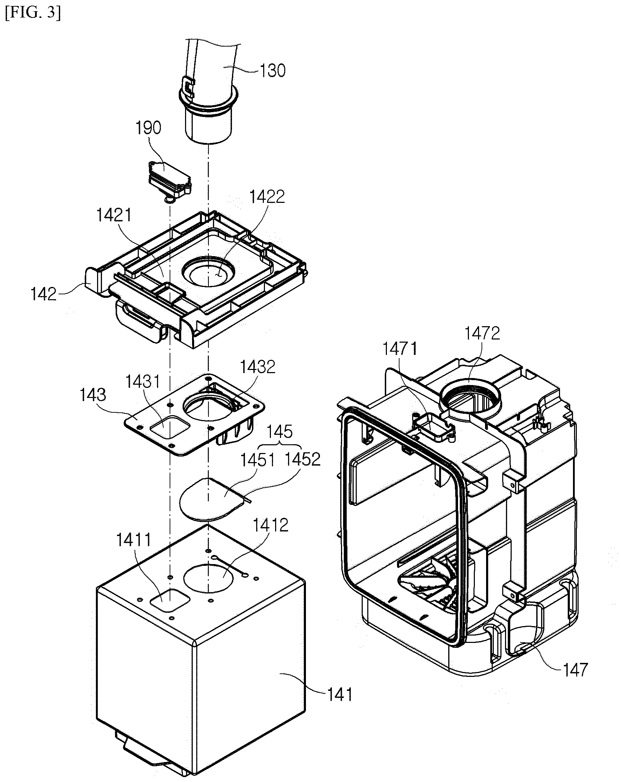

With reference to FIG. 3, the dust collecting body 141 is formed in a shape similar to a hexahedral shape. However, the present disclosure is not limited thereto. The dust collecting body 141 may be formed in other polyhedral or cylindrical shapes.

The dust inlet port 1412 is formed in the dust collecting body 141. The dust inlet port 1412 is formed at an upper side of the dust collecting body 141.

Alternatively, an upper side of the dust collecting body 141 may be opened, and an upper plate, in which the dust inlet port 1412 is formed, may be disposed at the upper side of the dust collecting body 141.

The dust inlet port 1412 of the dust collecting body 141 is disposed to overlap the dust inlet port 1422 of the outer upper plate 142 and the dust inlet port 1432 of the inner upper plate 143. Therefore, the dust inlet port 1412 of the dust collecting body 141 communicates with the dust inlet port 1422 of the outer upper plate 142 and the dust inlet port 1432 of the inner upper plate 143.

Hereinafter, the dust inlet ports 1412, 1422, and 1432 may include the dust inlet port 1412 of the dust collecting body 141, the dust inlet port 1422 of the outer upper plate 142, and the dust inlet port 1432 of the inner upper plate 143 as long as the dust inlet ports 1412, 1422, and 1432 do not conflict.

The dust collecting body 141 includes a spray module insertion hole 1411. At least a part of the spray module passes through the spray module insertion hole 1411.

The spray module insertion hole 1411 of the dust collecting body 141 is disposed to overlap the spray module installation part 1471 of the dust collecting part casing 147, a spray module insertion hole 1421 of the outer upper plate 142, and a spray module insertion hole 1431 of the inner upper plate 143 in the upward/downward direction.

The upper plates 142 and 143 are disposed on an upper surface of the dust collecting part 140. The dust inlet ports 1422 and 1432 are formed at one side of the upper plate.

The upper plates include the outer upper plate 142 and the inner upper plate 143.

The outer upper plate 142 is disposed on an outer surface of an upper wall of the dust collecting body 141. The outer upper plate 142 may be disposed to be perpendicular to the suction flow path 130.

The outer upper plate 142 includes the dust inlet port 1422. The dust inlet port 1422 is a hole formed through the outer upper plate 142 in the upward/downward direction. The dust inlet port 1422 of the outer upper plate 142 communicates with the dust inlet port 1432 of the inner upper plate 143 and the dust inlet port 1412 of the dust collecting body 141. The suction flow path 130 may be inserted into the dust inlet port 1422 of the outer upper plate 142.

The outer upper plate 142 includes the spray module insertion hole 1421. At least a part of the spray module is inserted into the spray module insertion hole 1421.

The spray module insertion hole 1421 of the outer upper plate 142 is disposed to overlap the spray module installation part 1471 of the dust collecting part casing 147, the spray module insertion hole 1411 of the dust collecting body 141, and the spray module insertion hole 1431 of the inner upper plate 143 in the upward/downward direction.

The inner upper plate 143 is disposed on an inner surface of the upper wall of the dust collecting body 141. The inner upper plate 143 may be disposed to be perpendicular to the suction flow path 130.

The inner upper plate 143 includes the dust inlet port 1432. The dust inlet port 1432 is a hole formed through the inner upper plate 143 in the upward/downward direction. The dust inlet port 1432 of the inner upper plate 143 communicates with the dust inlet port 1422 of the outer upper plate 142 and the dust inlet port 1412 of the dust collecting body 141.

The inner upper plate 143 includes the spray module insertion hole 1431. At least a part of the spray module passes through the spray module insertion hole 1431.

The spray module insertion hole 1431 of the inner upper plate 143 is disposed to overlap the spray module installation part 1471 of the dust collecting part casing 147, the spray module insertion hole 1411 of the dust collecting body 141, and the spray module insertion hole 1421 of the outer upper plate 142 in the upward/downward direction.

The inlet pipe 144 is disposed on the inner upper plate 143.

The inlet pipe 144 is a component configured to communicate with the suction flow path 130 and guide dust, which flows along the suction flow path 130, into the dust collecting body 141.

The inlet pipe 144 extends inward from peripheral surfaces of the dust inlet ports 1412, 1422, and 1432 and defines a circle of curvature so that an end of the inlet pipe 144 has a particular curvature when viewed from one side. Specifically, the inlet pipe 144 extends downward from the dust inlet port 1432 of the inner upper plate 143.

The inlet pipe cover 145 is a component configured to open or close the inlet pipe 144.

The inlet pipe cover 145 is disposed at the end of the inlet pipe 144.

One end of the inlet pipe cover 145 is rotatably coupled to the inlet pipe 144, and the other end of the inlet pipe cover 145 opens or closes the inlet pipe 144 while rotating about one end of the inlet pipe cover 145 as a rotation center.

The inlet pipe cover 145 may be moved by the dust collecting motor 150. For example, the dust collecting motor 150 is disposed below the inlet pipe cover 145 and provides negative pressure to the inlet pipe cover 145, such that the inlet pipe cover 145 may move downward and open the inlet pipe 144.

The cleaner station 100 includes the spray module 190. The spray module 190 is a component configured to kill insects existing in the dust collecting part 140 by spraying at least any one of a sterilization gas and particulates into the dust collecting part 140.

Insects such as spider mites, dust mites, and the like prefer high-temperature, high-humidity environments. For example, it is known that spider mites prefer a temperature of 25 to 30°C and a relative humidity of 60% or higher. This environment seasonally corresponds to late spring, early summer, or a rainy season.

For example, newly born spider mites may breed after about five days. In case that the dust bin 220 of the cleaner 100 is frequently emptied in the related art, there is no great problem because the dust bin is emptied before the breeding period of spider mites is not reached. However, according to the present disclosure, because the dust collecting part 140 of the cleaner station 100 is not emptied in about one month or longer, there is a problem in that spider mites may sufficiently grow. Therefore, the cleaner station 100 according to the present disclosure is equipped with the spray module 190 to suppress the breeding of insects, such as spider mites or dust mites, and kill insects.

The spray module 190 may be equipped with a plasma discharge device. The plasma discharge device may be an ion generation device configured to generate ions or an ozone generation device configured to generate ozone.

The ion generation device refers to a device configured to generate ions by means of plasma discharge or the like by generating high voltages. The ion generation device is a widely available device that may be readily operated by those skilled in the art, and a detailed description thereof will be omitted.

The ozone generation device refers to a device configured to generate ozone by means of plasma discharge or the like by generating high voltages. The ozone generation device is a widely available device that may be readily operated by those skilled in the art, and a detailed description thereof will be omitted.

The spray module 190 produces at least any one of the sterilization gas and particulates and sprays at least any one of the sterilization gas and particulates to the dust collecting part 140. The sterilization gas is a gas that is deadly to small insects such as spider mites or dust mites. The sterilization gas is produced by the spray module 190 and sprayed to the dust collecting part 140.

The sterilization gas contains fatal particulates (particles) deadly to insects. Hereinafter, unless otherwise specifically discussed about particulates, it is assumed that particulates are contained in the sterilization gas or sprayed into the dust collecting part together with the sterilization gas.

For example, the sterilization gas may contain ozone particles. According to the experiment, when dust mites are exposed to ozone with a concentration of 30 ppm or more for about three hours, nearly 100% of the dust mites are killed. It can be seen from the experiment that ozone is deadly to insects. The spray module 190 according to the present disclosure may produce ozone and spray the sterilization gas, which contains the produced ozone particles, to the dust collecting part 140, thereby killing insects.

As still another example, the sterilization gas may contain ion particles. According to the experiment, when dust mites are exposed to the produced anions for about 36 hours, about 95% of the dust mites are killed. In addition, when clothes and the like are exposed to anions, the number of mites is not increased any further on the exposed surface. It can be seen from the experiment that ions are deadly to insects. The spray module 190 according to the present disclosure may produce ions and spray at least any one of the sterilization gas and particulates containing the produced ion particles to the dust collecting part 140, thereby killing insects.

The ions discharged from the spray module 190 may come into contact with insects and kill the insects by means of an electric shock.

Alternatively, the ions discharged from the spray module 190 may come into contact with insects and impart polarities to the insects, thereby capturing the insects on electrode plates disposed at one side by using an electrical attractive force.

The spray module 190 is disposed at one side of the dust collecting part 140.

The spray module 190 is coupled to one side of the dust collecting part casing 147. The spray module 190 at least any one of the sterilization gas and particulates into the dust collecting body 141.

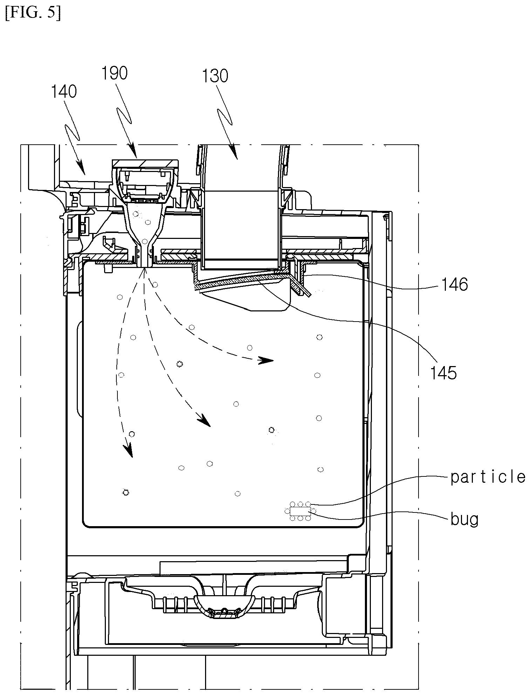

With reference to FIG. 5, the spray module 190 is disposed on the upper surface of the dust collecting part 140. The spray module 190 sprays the sterilization gas downward from the upper surface of the dust collecting body 141. The sterilization gas is diffused while being moved downward by gravity. Therefore, the sterilization gas may be diffused into the internal space of the dust collecting part 140 without a separate diffusion device such as a blower fan.

Unlike the configuration illustrated in FIG. 5, the spray module 190 may be disposed at a lateral or lower surface of the dust collecting part 140.

The sprayed sterilization gas contains particulates (particles). The particulates may come into contact with surfaces of insects while freely moving in the internal space of the dust collecting part 140. The insects may be killed as the particulates come into contact with the surfaces of the insects.

One end of the inlet pipe cover 145 may be rotatably coupled to the inlet pipe 144 or the dust collecting body 141, and the other end of the inlet pipe cover 145 may open or close the inlet pipe 144 while moving. In this case, a distance between the spray module 190 and one end of the inlet pipe cover may be longer than a distance between the spray module 190 and the other end of the inlet pipe cover. When the inlet pipe 144 is opened, the other end of the inlet pipe cover 145 is separated from the inlet pipe 144 first, and insects disposed at the other end may escape from the dust collecting part 140 first. Therefore, the spray module 190 may be disposed to be close to the other end of the inlet pipe cover 145 and concentratedly kill insects existing at the other end, thereby minimizing the escape of insects.

When the dust collecting body 141 is installed in the dust collecting part casing 147, at least a part of the spray module 190 is inserted into the dust collecting body 141.

The spray module 190 is installed in the dust collecting part casing 147 instead of the dust collecting body 141. Further, when the dust collecting body 141 is installed in the dust collecting part casing 147, at least a part of the spray module 190 is inserted into the dust collecting body 141, such that the sterilization gas may be sprayed into the dust collecting part 140. Therefore, the spray module 190 sprays the sterilization gas into the dust collecting body 141 only when the dust collecting body 141 is properly coupled to the dust collecting part casing 147, thereby preventing a leak of the sterilization gas.

The dust collecting body 141 is inserted into the dust collecting part casing 147 while moving in one direction. When the dust collecting body 141 moves in a direction intersecting the insertion direction, the dust collecting body 141 is fixed to the dust collecting part casing 147.

With reference to FIG. 5, the left side in the drawing is a front side of the cleaner station 100, and the right in the drawing is a rear side of the cleaner station 100. That is, the dust collecting body 141 is inserted into the dust collecting part casing 147 while moving from the front side toward the rear side.

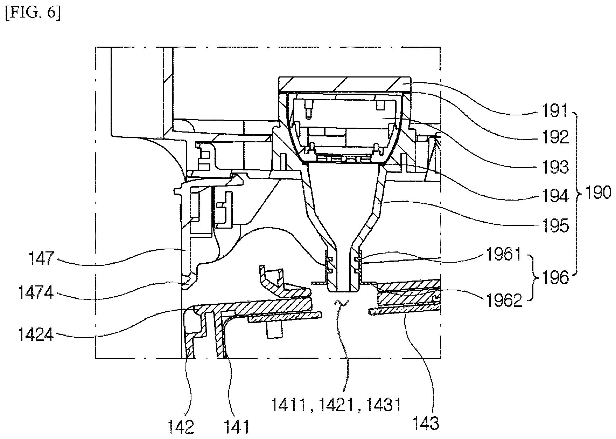

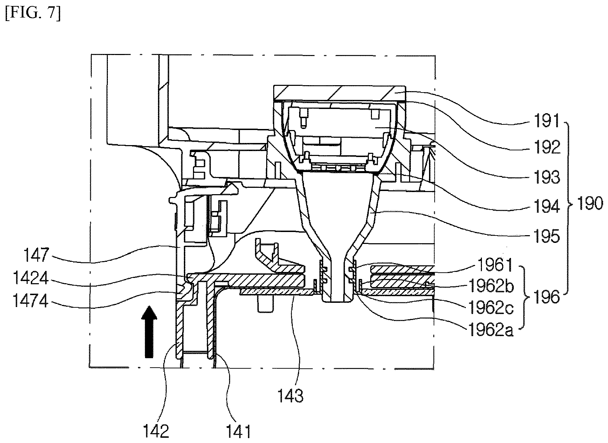

With reference to FIGS. 6 and 7, a rear end of the dust collecting body 141 is caught by one side of the dust collecting part casing 147, and a coupling hook 1424 of the outer upper plate 142 is caught by a corresponding hook 1474 of the dust collecting part casing 147 as a front end of the dust collecting body 141 moves upward, such that the dust collecting body 141 is fixed to the dust collecting part casing 147.

Because the direction in which the dust collecting body 141 is inserted into the dust collecting part casing 147 intersects the direction in which the dust collecting body 141 is fixed to the dust collecting part casing 147, the sterilization gas may be sprayed only when the dust collecting body 141 is properly coupled to the dust collecting part casing 147. In case that the direction in which the dust collecting body 141 is inserted into the dust collecting part casing 147 is parallel to the direction in which the dust collecting body 141 is fixed to the dust collecting part casing 147, the sterilization gas may be sprayed even when the dust collecting body 141 is less inserted, and the sterilization gas may be sprayed even when the dust collecting body 141 is slightly withdrawn. In contrast, according to the present disclosure, the direction in which the dust collecting body 141 is inserted into the dust collecting part casing 147 intersects the direction in which the dust collecting body 141 is fixed to the dust collecting part casing 147, such that whether the dust collecting body 141 is properly coupled to the dust collecting part casing 147 may be identified, and a leak of the sterilization gas may be prevented.

The spray module 190 includes an upper cover 191, an upper sealer 192, a sterilization gas generator 193, a spray guide 195, a lower sealer 194, and a guide sealer 196.

The spray module 190 includes the upper cover 191. The upper cover 191 is a component configured to constitute an upper surface of the spray module 190 and cover an upper surface of the sterilization gas generator 193.

The upper cover 191 is formed in a plate shape.

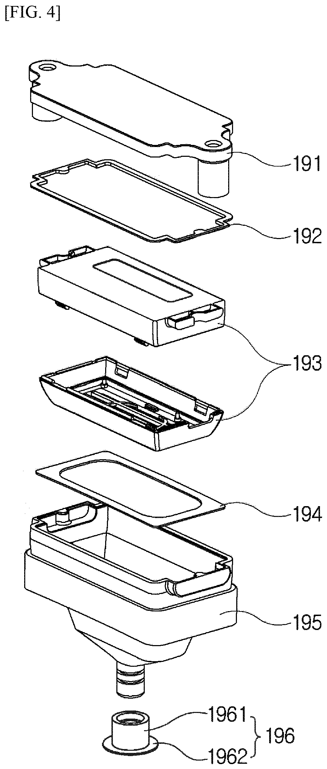

Fastening parts may be disposed at one side of the upper cover 191 and fixed to the dust collecting part casing 147. With reference to FIG. 4, the fastening parts may be disposed at two opposite ends of the upper cover 191.

The spray module 190 includes the upper sealer 192. The upper sealer 192 is a component that fills a gap between the upper cover 191 and the spray guide 195.

A lower surface of the upper sealer 192 is tightly attached to an upper surface of the spray guide 195, and an upper surface of the upper sealer 192 is tightly attached to the upper cover 191.

The upper sealer 192 may be made of a material, such as rubber, having flexibility.

The spray module 190 includes the sterilization gas generator 193. The sterilization gas generator 193 is a component configured to produce at least any one of the sterilization gas and particulates.

The sterilization gas generator 193 is disposed in the dust collecting part casing 147. The sterilization gas generator 193 is disposed in the dust collecting part casing 147 instead of the dust collecting body 141, such that foreign substances do not penetrate into the sterilization gas generator 193 at the time of washing the dust collecting body 141.

The spray guide has a space therein, and the sterilization gas generator 193 is disposed in the internal space of the spray guide.

The sterilization gas generator 193 may produce ozone. The sterilization gas generator 193 may be an ozone generation device. That is, the sterilization gas generator 193 may produce ozone by means of plasma discharge or the like by generating high voltages.

The ozone produced by the sterilization gas generator 193 may be sprayed into the dust collecting body 141, thereby killing insects.

The sterilization gas generator 193 may produce ions. The sterilization gas generator 193 may be an ion generation device. That is, the sterilization gas generator 193 may produce ions by means of plasma discharge or the like by generating high voltages.

The ions produced by the sterilization gas generator 193 may be sprayed into the dust collecting body 141 to kill insects by means of an electric shock or impart polarities to insects to capture the insects on the electrode plates.

The spray module 190 includes the spray guide 195. The spray guide is a component configured to guide the sterilization gas into the dust collecting part 140. The spray guide 195 is disposed at an outlet end of the sterilization gas generator 193.

The spray guide 195 has a space therein. An upper side of the spray guide 195 is opened. Therefore, the sterilization gas generator 193 is inserted through the upper side of the spray guide 195 from above the spray guide 195 and accommodated in the internal space of the spray guide 195. Thereafter, the upper side of the spray guide 195 is covered by the upper cover 191 and sealed by the sterilization gas generator 193.

A stepped portion is formed in the spray guide 195. A lower end of the sterilization gas generator 193 is caught and fixed by the stepped portion. The lower sealer 194 may be disposed on the stepped portion.

An outlet end is formed on a lower portion of the spray guide 195. The outlet end is formed in a shape having a wide upper side and a narrow lower side. Therefore, the particulates are sprayed downward through the outlet end of the spray guide 195.

The sterilization gas generator 193 is disposed in the dust collecting part casing 147, the spray guide 195 extends toward the dust collecting body 141, and the outlet end of the spray guide 195 is inserted into the dust collecting body 141. Therefore, the sterilization gas generator 193 and the dust collecting body 141 are spaced apart from each other by a predetermined distance. Therefore, it is possible to prevent insects from entering the sterilization gas generator 193 and prevent the collected dust from reversely flowing into the sterilization gas generator 193, thereby preventing a breakdown of the sterilization gas generator 193.

The spray module 190 includes the lower sealer 194. The lower sealer 194 is a component that fills a gap between the sterilization gas generator 193 and the spray guide 195.

A lower surface of the lower sealer 194 is tightly attached to the stepped portion of the spray guide 195, and an upper surface of the lower sealer 194 is tightly attached to a lower surface of the sterilization gas generator 193.

The lower sealer 194 may be made of a material, such as rubber, having flexibility.

The upper sealer 192 and the lower sealer 194 may doubly seal the gap through which the sterilization gas may leak, thereby more assuredly preventing a problem of a leak of the sterilization gas.

A direction in which the sterilization gas is sprayed by the spray module 190 may be parallel to a direction in which air is introduced into the outlet end of the suction flow path. With reference to FIG. 5, the sterilization gas is sprayed downward from the spray module 190, and the air is also introduced into the outlet end of the suction flow path. As described above, the sterilization gas is naturally diffused without a component separately provided to diffuse the sterilization gas. Because the direction in which the air is introduced into the suction flow path is parallel to the direction in which the sterilization gas is sprayed, the sterilization gas is prevented from reversely flowing at the outlet end of the spray guide 195, and the sterilization gas is more easily sprayed on the basis of the Bernoulli's theorem principle.

The spray module 190 includes the guide sealer 196. The guide sealer 196 is a component that fills a gap between the spray module guide and the dust collecting part 140.

The guide sealer 196 is disposed at an outlet end of the spray module 190. Specifically, the guide sealer 196 is disposed at the outlet end of the spray guide 195.

The guide sealer 196 is coupled to an outer peripheral surface of the outlet end of the spray module 190. An annular groove may be formed in an outer peripheral surface of the outlet end of the spray module 190. An annular protruding portion may be formed on an inner peripheral surface of the guide sealer 196. The protruding portion is inserted into the groove, such that the guide sealer 196 may be coupled to the spray module 190.

The guide sealer 196 is disposed between the outlet end of the spray module 190 and the dust collecting part 140. The outlet end of the spray module 190 is disposed inside the guide sealer 196, and the dust collecting part 140 is disposed on an outer peripheral surface of the guide sealer 196.

A length from one end to the other end of the guide sealer 196 may be longer than a length from one end to the other end of the spray module insertion hole 1411. With reference to FIGS. 6 and 7, the length from one end to the other end of the guide sealer 196 indicates a diameter of the guide sealer 196. The length from one end to the other end of the spray module insertion hole 1411 indicates a diameter of the spray module insertion hole 1411. With reference to FIG. 6, the diameter of the guide sealer 196 is larger than the diameter of the spray module insertion hole 1411 of the dust collecting body 141. Therefore, as illustrated in FIG. 7, when the spray guide 195 is inserted into the dust collecting body 141, one side of the guide sealer 196 is bent. Therefore, a gap between the spray guide 195 and the dust collecting body 141 may be sealed.

The guide sealer 196 may include a coupling portion 1961 and a sealing portion 1962.

The coupling portion 1961 is a component coupled to the spray guide 195.

The coupling portion 1961 is coupled to the outer peripheral surface of the outlet end of the spray module 190. An annular protruding portion may be formed on an inner peripheral surface of the coupling portion 1961 and inserted into the annular groove formed in the outer peripheral surface of the outlet end of the spray module 190.

The sealing portion 1962 is a component that fills the gap between the spray guide 195 and the dust collecting body 141.

The sealing portion 1962 protrudes radially outward from the coupling portion 1961. Specifically, the sealing portion 1962 may protrude radially outward from a lower end of the coupling portion 1961.

The sealing portion 1962 may include a non-deformable portion 1962a, a deformable portion 1962b, and a bent portion 1962c.

The non-deformable portion 1962a is a portion of the sealing portion 1962 that is not deformed.

The non-deformable portion 1962a extends outward from the spray guide 195 and is disposed adjacent to the dust collecting part 140.

A diameter of the non-deformable portion 1962a may be equal to the diameter of the spray module insertion hole 1411 of the dust collecting body 141.

The deformable portion 1962b is a portion of the sealing portion 1962 that is bent and deformed.

The deformable portion 1962b further extends outward from an end of the non-deformable portion 1962a and is at least partially deformed by being tightly attached to the dust collecting part 140.

The bent portion 1962c is disposed between the deformable portion 1962b and the non-deformable portion 1962a. The bent portion 1962c is disposed between an outer peripheral surface of the non-deformable portion 1962a and an inner peripheral surface of the deformable portion 1962b.

With reference to FIG. 7, the deformable portion 1962b may extend upward based on the bent portion 1962c.

The sealing portion 1962 may include the non-deformable portion 1962a horizontally extending based on the bent portion 1962c, and the deformable portion 1962b vertically extending based on the bent portion 1962c, thereby more effectively sealing the gap between the spray guide 195 and the dust collecting part 140.

When the spray module 190 operates for a preset reference operation time, the operation of the spray module 190 is stopped for a preset reference pause time or longer. In case that the dust collecting motor operates, dust is introduced into the cleaner station 100 through the coupling portion 1961, passes through the dust collecting part 140, and is discharged through a discharge port (not illustrated). In this case, ions or ozone remaining in the dust collecting part 140 may be discharged to the outside of the cleaner station 100, and the discharged ions or ozone may harm the user. Therefore, a sufficient pause time is required so that the ion or ozone is decomposed. Therefore, the cleaner station 100 according to the present disclosure provides a pause time equal to or longer than a reference pause time so that the ion or ozone in the sterilization gas is sufficiently decomposed.

When there is a dust suction instruction for capturing dust in the dust bin of the cleaner 100, the dust collecting motor may operate after the reference pause time elapses.

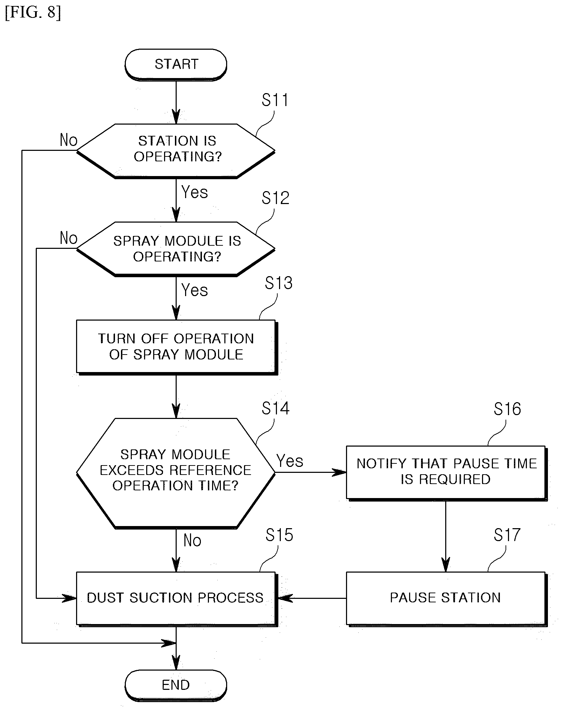

FIG. 8 illustrates an operation S10 of the spray module 190 in an operating mode in which the cleaner station 100 sucks dust in the cleaner 100.

A control unit 400 may include a printed circuit board and elements mounted on the printed circuit board. The control unit is disposed in the cleaner station 100. The control unit may be disposed in the cleaner 100. The control unit may control the components of the cleaner station 100. Alternatively, the control unit may also control the components of the cleaner.

The control unit determines whether the cleaner station 100 is operating (S11). The operation of the cleaner station 100 may include a series of steps for collecting dust in the cleaner 100. Specifically, the present step may be a preparation step that is performed previously before a dust suction step S 15 to be described below.

The control unit ends the step when the cleaner station 100 is not operating. In this case, the control unit may perform step S20.

In case that the cleaner station 100 is operating, the control unit determines whether the spray module 190 is operating (S12).

In case that the spray module 190 is operating, the control unit stops the operation of the spray module 190 (S13). The sterilization gas exists in the dust collecting part 140 in the state in which the spray module 190 is operating. When the dust collecting motor operates in this state, the harmful sterilization gas may be discharged to the outside of the cleaner station 100. Therefore, the control unit stops the operation of the spray module 190 before a dust suction process is performed.

In case that the spray module 190 is in the stopped state, the control unit initiates the dust suction process (S 15). In case that the spray module 190 does not operate, the sterilization gas does not exist in the dust collecting part 140. Therefore, there is no concern that the sterilization gas is discharged to the outside of the cleaner station 100 even though the dust suction process is performed immediately. Therefore, the control unit may immediately perform the dust suction process.

After the operation of the spray module 190 is stopped in step S13, the control unit identifies whether the spray module 190 exceeds the reference operation time (S14).

In case that the spray module 190 operates for a time shorter than the reference operation time, the control unit determines that ion or ozone exists in an allowable range in the dust collecting part 140. Therefore, the control unit immediately initiates the dust suction process (S 15).

In case that the spray module 190 operates beyond the reference operation time, the control unit determines that ion or ozone exists in the allowable range or more in the dust collecting part 140. Therefore, the control unit notifies the user that a pause time is required (S16), and the control unit pauses the cleaner station 100 for the reference pause time (S17). The control unit initiates the dust suction process after the reference pause time elapses (S15).

The reference operation time may be a value pre-stored in the control unit or a storage part. For example, the reference operation time may be 30 minutes. In other words, the pause time is performed in case that the spray module 190 has operated for more than 30 minutes. The dust suction process is performed immediately in case that the spray module 190 has operated for less than 30 minutes.

The reference pause time may be a value pre-stored in the control unit or the storage part. For example, the reference operation time may be 15 minutes.

In the operating mode, there is a concern that the sterilization gas leaks to the outside of the cleaner station 100. According to the present disclosure, the sufficient time is ensured so that the sterilization gas is decomposed before the dust suction process is performed. Therefore, it is possible to minimize a risk of harm to the user caused by a leak of the sterilization gas.

The spray module 190 may alternately operate and stop operating while the dust collecting motor does not operate.

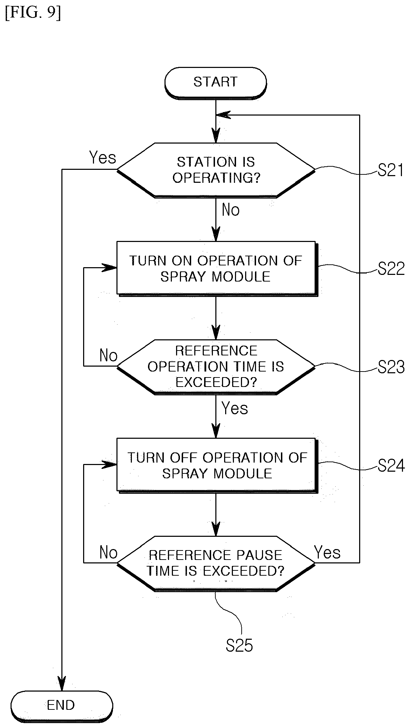

FIG. 9 illustrates an operation S20 of the spray module 190 in a stop mode in which the cleaner station 100 does not operate.

The control unit determines whether the cleaner station 100 is operating (S21). The operation of the cleaner station 100 may include a series of steps for collecting dust in the cleaner 100. In other words, the present step refers to a step in which the cleaner station 100 is on standby after the cleaner station 100 sucks overall dust in the cleaner 100.

The control unit ends the step in case that the cleaner station 100 is operating. In this case, the control unit may perform step S10 described above.

In case that the cleaner station 100 is not operating, the control unit operates the spray module 190 (S22). When the spray module 190 operates, the sterilization gas is sprayed into the dust collecting part 140 and kills insects.

The control unit operates the spray module 190 for the reference operation time. After a predetermined time has elapsed, the control unit identifies whether the reference operation time has elapsed after the spray module 190 has operated (S23). In case that the reference operation time has not elapsed, the control unit operates the spray module 190 until the reference operation time elapses.

After the reference operation time elapses, the control unit stops the operation of the spray module 190 (S24). In case that the operation of the spray module 190 is stopped, the sterilization gas is not introduced into the dust collecting part 140 any further.

After the predetermined time has elapsed, the control unit identifies whether the reference pause time has elapsed (S25). In case that the reference pause time has not elapsed, the control unit stops the spray module 190 until the reference pause time elapses.

The control unit performs step S21 again after the reference pause time elapses. The control unit may repeatedly perform steps S21 to S25 while the cleaner station 100 does not operate again.

The reference operation time may be a value pre-stored in the control unit or the storage part. For example, the reference operation time may be 3 hours. In other words, the pause step is performed in case that the spray module 190 has operated beyond 3 hours.

The reference pause time may be a value pre-stored in the control unit or the storage part. For example, the reference operation time may be 6 hours.

There is no concern in the stop mode that the sterilization gas leaks to the outside of the cleaner station 100. However, it is necessary to prevent the breeding of insects with optimal power consumption. According to the present disclosure, the spray module 190 may alternately operate and stop operating at a particular time interval, thereby preventing the breeding of insects with minimum power consumption.

An operational effect of the cleaner station 100 according to the present disclosure configured as described above will be described.

According to the present disclosure, the spray module 190 may be disposed at one side of the dust collecting part 140 and spray the sterilization gas into the dust collecting part 140 to kill insects existing in the dust collecting part 140. Therefore, it is possible to maintain the cleanliness of the interior of the dust collecting part 140.

According to the present disclosure, the spray module 190 is disposed in the dust collecting part casing 147 and sprays the sterilization gas into the dust collecting body 141. The dust collecting body 141 may be separated from the dust collecting part casing 147 and washed with water or the like. Because the spray module 190 is disposed in the dust collecting part casing 147 instead of the dust collecting body 141, which may prevent water from penetrating into the spray module 190 and causing a breakdown of the spray module 190.

According to the present disclosure, when the dust collecting body 141 is installed in the dust collecting part casing 147, at least a part of the guide sealer 196 is deformed and fills the gap between the dust collecting body 141 and the spray module guide, which may prevent a leak of the sterilization gas.

In the operating mode, there is a concern that the sterilization gas leaks to the outside of the cleaner station 100. According to the present disclosure, the sufficient time is ensured so that the sterilization gas is decomposed before the dust suction process is performed. Therefore, it is possible to minimize a risk of harm to the user caused by a leak of the sterilization gas.

There is no concern in the stop mode that the sterilization gas leaks to the outside of the cleaner station 100. However, it is necessary to prevent the breeding of insects with optimal power consumption. According to the present disclosure, the spray module 190 may alternately operate and stop operating at a particular time interval, thereby preventing the breeding of insects with minimum power consumption.

While the exemplary embodiments of the present disclosure have been illustrated and described above, the present disclosure is not limited to the specific exemplary embodiments, and various modifications can of course be made by those skilled in the art to which the present disclosure pertains without departing from the subject matter of the present disclosure as claimed in the claims. Further, the modifications should not be appreciated individually from the technical spirit or prospect of the present disclosure.

### [Description of Reference Numerals]

10: Cleaner system
100: Cleaner station
200: Cleaner
110: Housing
120: Coupling part
130: Suction flow path
140: Dust collecting part
141: Dust collecting body
142: Outer upper plate
143: Inner upper plate
144: Inlet pipe
1444: Corresponding member
145: Inlet pipe cover
147: Dust collecting part casing
190: Spray module
191: Upper cover
192: Upper sealer
193: Sterilization gas generator
194: Spray guide
195: Lower sealer
196: Guide sealer
1961: Coupling portion
1962: Sealing portion
1962a: Non-deformable portion
1962b: Deformable portion
1962c: Bent portion

## Claims

1. A cleaner station comprising:
a suction flow path having an inlet end configured to communicate with a dust bin of a cleaner;
a dust collecting part connected to an outlet end of the suction flow path and configured to capture dust in the dust bin of the cleaner; and
a spray module disposed at one side of the dust collecting part and configured to spray at least any one of the sterilization gas and particulates into the dust collecting part to kill insects existing in the dust collecting part.

2. The cleaner station of claim 1, wherein the dust collecting part comprises:
a dust collecting part casing configured to define a space therein; and
a dust collecting body separably installed in the dust collecting part casing, and
wherein the spray module is installed at one side of the dust collecting part casing and sprays at least any one of the sterilization gas and particulates into the dust collecting body.

3. The cleaner station of claim 2, wherein when the dust collecting body is installed in the dust collecting part casing, at least a part of the spray module is inserted into the dust collecting body.

4. The cleaner station of claim 3, wherein the dust collecting body is inserted into the dust collecting part casing while moving in one direction and fixed to the dust collecting part casing while moving in a direction intersecting the direction in which the dust collecting body is inserted.

5. The cleaner station of claim 1, wherein the spray module comprises a guide sealer disposed at the outlet end and configured to fill a gap between the outlet end and the dust collecting part.

6. The cleaner station of claim 5, wherein the dust collecting part comprises a spray module insertion hole penetrated by the spray module, and a length from one end to the other end of the guide sealer is longer than a length from one end to the other end of the spray module insertion hole.

7. The cleaner station of claim 5, wherein the guide sealer comprises:
a non-deformable portion extending outward from the spray guide and disposed adjacent to the dust collecting part; and
a deformable portion further extending outward from an end of the non-deformable portion and configured to be at least partially deformed by being tightly attached to the dust collecting part.

8. The cleaner station of claim 1, wherein the spray module comprises:
a sterilization gas generator configured to produce at least any one of the sterilization gas and particulates; and
a spray guide disposed at an outlet end of the sterilization gas generator and configured to guide at least any one of the sterilization gas and particulates into the dust collecting part.

9. The cleaner station of claim 8, wherein the dust collecting part comprises:
a dust collecting part casing configured to define a space therein; and
a dust collecting body separably installed in the dust collecting part casing,
wherein the sterilization gas generator is disposed in the dust collecting part casing,
wherein the spray guide extends toward the dust collecting body, and
wherein an outlet end of the spray guide is inserted into the dust collecting body.

10. The cleaner station of claim 8, wherein the sterilization gas generator produces ozone.

11. The cleaner station of claim 8, wherein the sterilization gas generator produces ions.

12. The cleaner station of claim 1, wherein a direction in which at least any one of the sterilization gas and particulates is sprayed by the spray module is parallel to a direction in which air is introduced into the outlet end of the suction flow path.

13. A cleaner station comprising:
a suction flow path having an inlet end configured to communicate with a dust bin of a cleaner;
a dust collecting part connected to an outlet end of the suction flow path and configured to capture dust in the dust bin of the cleaner; and
a spray module disposed at one side of the dust collecting part and configured to spray at least any one of the sterilization gas and particulates into the dust collecting part to kill insects existing in the dust collecting part,
wherein an operation of the spray module is stopped for a preset reference pause time or longer when the spray module operates for a preset reference operation time.

14. The cleaner station of claim 13, further comprising:
a dust collecting motor configured to generate an airflow in the suction flow path,
wherein when there is a dust suction instruction for capturing dust in the dust bin of the cleaner, the dust collecting motor operates after the reference pause time elapses.

15. The cleaner station of claim 13, further comprising:
a dust collecting motor configured to generate an airflow in the suction flow path,
wherein the spray module alternately operates and stops operating while the dust collecting motor does not operate.
